# EUROPEAN PATENT SPECIFICATION

(11) **EP 0 339 964 B1**
(45) Date of publication and mention of the grant of the patent: **14.12.1994**
(21) Application number: 89304150.9
(22) Date of filing: 26.04.1989
(51) Int. Cl.: C07C 337/00

(54) **Improved synthesis of 4-methyl-3-thiosemicarbazide**
Synthese von 4-Methyl-3-thiosemicarbazid
Synthèse de 4-methyl-3-thiosemicarbazide

(30) Priority: 27.04.1988 US 185632
(43) Date of publication of application: 02.11.1989
(73) Proprietor: DowElanco, Indianapolis, Indiana 46268-1189 (US)
(72) Inventor: Achgill, Ralph Kenneth, Lafayette Indiana 47905 (US); Call, Laurence Wesley, Lafayette Indiana 47904 (US)
(74) Representative: Raynor, John

(56) References cited:
- EP-A- 0 308 225
- DE-A- 2 225 482
- DE-A- 2 546 096
- DE-C- 832 891
- US-A- 4 237 066
- CHEMICAL ABSTRACTS, vol. 95, 1981, page 716, abstract no. 62142t, Columbus, Ohio, US; I. BELAI et al.: "Tetrahydro-2-thioxo-2H-1,3,5-thiadiazin-4-ones and their derivatives representing a novel heterocyclic ring system"

## Description

This invention relates to an improved synthesis of 4-methyl-3-thiosemicarbazide (MTSC) and to novel methyldithiocarbamate quaternary salts of value in that synthesis. MTSC is a useful intermediate in the production of herbicides.

U.S. Patent Specifications Nos. 4,132,736 and 4,237,066 describe processes for preparing MTSC, but the present invention provides a process which is superior both in terms of yield and ease of recovery of reaction products.

Thus, in accordance with the present invention, there is provided a process for preparing MTSC: 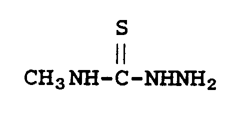
which comprises reacting a methyldithiocarbamate quaternary ammonium salt of Formula (I): 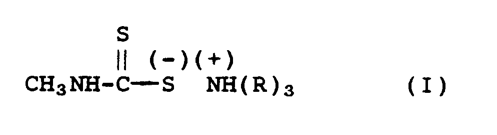
where R is C₁-C₄ alkyl, with hydrazine.

The reaction is preferably effected in an aqueous system, for example, using water as the sole solvent. However, mixtures of water with a lower alkanol such as ethanol may also be utilized. Suitable reaction temperatures lie within the range 50 to 130° C, although a temperature of about 100° C is preferred since this is the boiling point of water, the most preferred solvent. It is also preferred to effect the reaction under an inert gas atmosphere such as nitrogen.

Usually, the R groups in the amino group -N(R)₃ will be the same, and in the preferred embodiment of the invention, R will be ethyl. In this context, the reaction of the invention can be schematically illustrated. 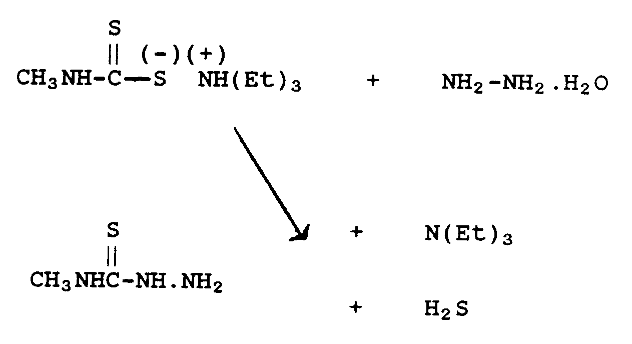
Triethylamine is a liquid at room temperature and can be trapped in a receiver and easily recovered. This is to be contrasted with the amine products of the art reactions which are mono or dialkylamines which are very hard to recover since they have low boiling points and are soluble in the aqueous phase. The triethylamine also assists with removal of H₂S and helps with control of the reaction temperature.

The hydrazine will normally be utilized as the hydrate although, if appropriate care is taken, other forms such as acid-addition salts, for example, the hydrochloride, may be utilized.

The methyldithiocarbamate quaternary ammonium salt can be conveniently prepared in situ by reaction of approximately equimolar amounts of carbon disulphide, methylamine and trialkylamine. Thus: 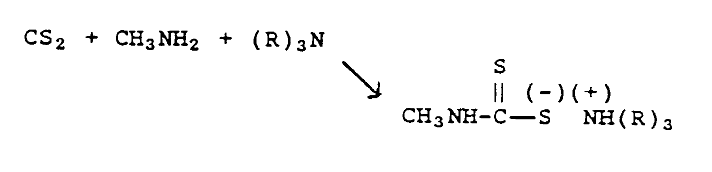
Preferably R is ethyl.

This reaction is preferably accomplished in polar solvents such as water. Preferred reaction temperatures are for example 25-35° C., at which temperatures, the reaction is substantially complete after about 3 hours.

As mentioned above, MTSC is a useful intermediate in the herbicidal field. Thus, for example, MTSC can be reacted with pivalic acid in the presence of polyphosphoric acid to yield 2-(t-butyl)-5-methylamine-1,3,4-thiadiazole. That material can be reacted with 1,3-dimethylurea in the presence of HCl to yield the well known herbicide tebuthiuron.

The invention will now be further illustrated with reference to the following non-limiting Examples.

### Example 1

### 4-METHYL-3-THIOSEMICARBAZIDE

(A) Methylamine (41.5g) and deionized water (50 ml) were introduced into a 250 ml three-necked flask. A mixture of CS₂ (38 g) and triethylamine (50.5 g) was then added to the flask over a period of 1 hour while maintaining the reaction mixture at a temperature between 25 and 30° C. The reaction mixture was then stirred for a further hour to generate the methyl dithiocarbamate quaternary ammonium salt: 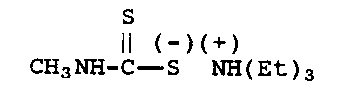 This material was not isolated but was used in situ in the next phase.
(B) Hydrazine hydrate (100%, 33 g) and deionized water (100 ml) were added to a 500 ml flask, and the aqueous mixture then heated to 102° C. The reaction mixture obtained in (A) (170 ml) was then added over 1 hour whilst distilling off an equal volume of water. An additional 130 ml of water was then added while simultaneously distilling off an equal volume of water. The reaction mixture was then cooled to 65° C and seeded with crystals of MTSC. After seeding, the reaction mixture was slowly cooled to 0° C, stirred for ½ hour and filtered. The filter cake was washed with 50 ml of deionized water, and dried in vacuo overnight.
   Yield of title product: 42 g (m.p. 131-135° C.)

### Example 2

The process of Example 1 was repeated except that the methyldithiocarbamate quaternary salt was isolated for characterization purposes.

The structure was confirmed by NMR, MS and IR. ¹H NMR (D₆-DMSO): δ 6.75 (m, 1H), 3.2 (q, 6H), 2.9 (d, 3H), 1.25 (t, 9H) MS identified a negative ion peak at 106 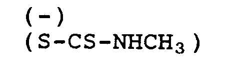
and positive ion scan showed a peak at 102

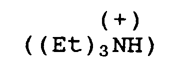

### Example 3

(A) To a 500 ml 3-necked flask was added 40% methylamine (93.8 g), deionized water (108 ml) and triethylamine (109.1 g). The contents of the flask were then thoroughly mixed. Carbon disulphide (82.1 g) was then added to the reaction mixture over a period of 1 hour, whilst maintaining the agitated reactants at a temperature of 30 to 35° C. The reaction mixture was then stirred for an additional hour at 30° C.
(B) To a different 500 ml 3-necked flask was added hydrazine hydrate (100%, 71.3 g) and deionized water (162 ml). This aqueous mixture was heated to 95-100° C. under an inert gas atmosphere.
(C) The reaction mixture from (A) was then added to the aqueous hydrazine hydrate system of (B) over a period of 3 hours whilst maintaining the reaction temperature in the range from 95 to 100° C.

The volume in the reaction vessel was then reduced by distillation for 1.5 hours.

Triethylamine was recovered via an attached scrubber containing aqueous caustic soda (50% NaOH, 172.8 g; 108 ml of deionized water). The triethylamine was distilled off by heating the mixture in the scrubber to 110° C.

The contents of the reaction flask were then cooled to 65° C. and seeded with crystals of MTSC. After crystallization of the product, the slurry was cooled to 0° C. and stirred for one-half hour. After filtration, the filter cake was washed with chilled deionized water, and then dried in vacuo at 60° C. overnight.

Yield of MTSC was 92.8 g (81.4%).

### Example 4

The procedure of Example 3 was repeated except that 100 ml of deionized water was used instead of 108 ml in Step (A). In addition, only 60 ml of deionized water was used in conjunction with the hydrazine hydrate in step (B).

Yield of MTSC was 98.8 g (86.1%).

## Claims

1. A process for preparing 4-methyl-3-thiosemicarbazide: 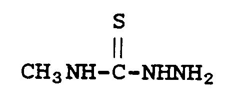 which comprises reacting a methyldithiocarbamate quaternary ammonium salt of Formula (I): 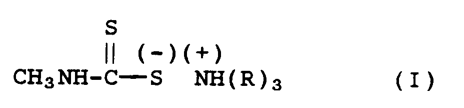 wherein R is C₁-C₄ alkyl, with hydrazine.

2. A process according to claim 1 carried out in an aqueous system.

3. A process according to claim 1 or 2, wherein R is ethyl.

4. A process according to any one of the preceding claims, wherein the methyldithiocarbamate quaternary ammonium salt is prepared in situ from the reaction of methylamine, carbon disulphide and the amine of formula (R)₃N.

5. A process according to any one of the preceding claims, wherein the hydrazine is in the form of hydrazine hydrate.

6. A process according to claim 1, carried out in an aqueous system, wherein R is ethyl, wherein the methyldithiocarbamate quaternary ammonium salt is prepared in situ from the reaction of methylamine, carbon disulphide and triethylamine and wherein the hydrazine is in the form of hydrazine hydrate.

## Patentansprüche

1. Verfahren zur Herstellung von 4-Methyl-3-thiosemicarbazid: 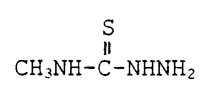 welches den folgenden Schritt umfaßt:
Umsetzen eines quaternären Ammoniumsalzes von Methyldithiocarbamat der Formel (I) 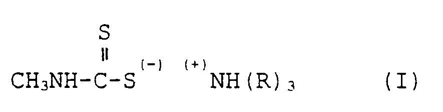 worin R C₁-C₄-Alkyl ist, mit Hydrazin.

2. Verfahren nach Anspruch 1, das in einem wäßrigen System durchgeführt wird.

3. Verfahren nach Anspruch 1 oder 2, wobei R Ethyl ist.

4. Verfahren nach einem der vorhergehenden Ansprüche, wobei das quaternäre Ammoniumsalz von Methyldithiocarbamat aus der Reaktion von Methylamin, Kohlenstoffdisulfid und dem Amin der Formel (R)₃N in situ hergestellt wird.

5. Verfahren nach einem der vorhergehenden Ansprüche, wobei das Hydrazin in Form von Hydrazinhydrat vorliegt.

6. Verfahren nach Anspruch 1, das in einem wäßrigen System durchgeführt wird, wobei R Ethyl darstellt, wobei das quaternäre Ammoniumsalz von Methyldithiocarbamat aus der Reaktion von Methylamin, Kohlenstoffdisulfid und Triethylamin in situ hergestellt wird und wobei das Hydrazin in Form von Hydrazinhydrat vorliegt.

## Revendications

1. Procédé pour la préparation de 4-méthyl-3-thiosemicarbazide: 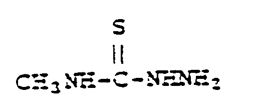 qui comprend la réaction d'un sel d'ammonium quaternaire d'un méthyldithiocarbamate de Formule (I): 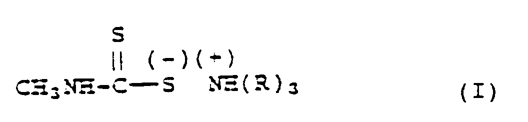 dans laquelle R représente un groupe alkyle en C₁-C₄, avec l'hydrazine.

2. Procédé selon la revendication 1, réalisé en milieu aqueux.

3. Procédé selon la revendication 1 ou 2, dans lequel R représente un groupe éthyle.

4. Procédé selon l'une quelconque des revendications précédentes, dans lequel le sel d'ammonium quaternaire du méthyldithiocarbamate est préparé in situ à partir de la réaction entre la méthylamine, le disulfure de carbone et l'amine de formule (R)₃N.

5. Procédé selon l'une quelconque des revendications précédentes, dans lequel l'hydrazine est sous forme d'hydrate d'hydrazine.

6. Procédé selon la revendication 1, réalisé en milieu aqueux, dans lequel R représente un groupe éthyle, dans lequel le sel d'ammonium quaternaire du méthyldithiocarbamate est préparé in situ à partir de la réaction entre la méthylamine, le disulfure de carbone et la triéthylamine et dans lequel l'hydrazine est sous forme d'hydrate d'hydrazine.
